# EUROPEAN PATENT SPECIFICATION

(11) **EP 3 168 763 B1**
(45) Date of publication and mention of the grant of the patent: **30.12.2020**
(21) Application number: 16173303.5
(22) Date of filing: 07.06.2016
(51) Int. Cl.: G16H 10/20

(54) **A MONITORING DEVICE AND A METHOD OF MONITORING DRUG ADHERENCE IN A CLINICAL TRIAL**
ÜBERWACHUNGSVORRICHTUNG UND VERFAHREN ZUR ÜBERWACHUNG DER EINHALTUNG VON ARZNEIMITTELDOSEN IN EINER KLINISCHEN STUDIE
DISPOSITIF DE SURVEILLANCE ET PROCÉDÉ DE SURVEILLANCE DE L'ADHÉRENCE D'UN MÉDICAMENT DANS UN ESSAI CLINIQUE

(30) Priority: 13.11.2015 IN 6118CH2015
(43) Date of publication of application: 17.05.2017
(73) Proprietor: Wipro Limited, 560 035 Karnataka (IN)
(72) Inventor: PATHANGAY, Vinod, 560100 Karnataka (IN); MADATHIL, Santhosh Kumar, 560035 Karnataka (IN)
(74) Representative: Finnegan Europe LLP

(56) References cited:
- US-A1- 2006 061 472
- US-A1- 2006 210 626
- US-A1- 2014 009 262
- US-B1- 9 047 746

## Description

### Field of the disclosure

The present subject matter is related, in general to monitoring system, and more particularly, but not exclusively to a monitoring device and a method of monitoring drug adherence in a clinical trial.

### Background

Healthcare remains one of the ever evolving and ever demanding fields in pursuit of healthiness. There are numerous ailments that seem to affect the human beings. Many of these ailments have been detected a long time ago but they are not yet treatable. On the other hand, many newer ailments seem to surface every other day. In the endeavor to treat such ailments and improve upon the existing ones pharmaceutical companies, researchers and scientists invent, experiment and test various chemicals to come up with new molecules and drugs for treating these ailments. However, the new molecules and drugs are not directly permitted to be consumed by humans for the obvious reason that these drugs need to be validated using a large scale clinical trial.

In a typical clinical trial, a number of volunteers are enrolled and are prescribed with dosages of particular drug, in the form of tablets or capsules, for predefined time duration. This is done in order to verify the effectiveness of the drug for the treating a particular condition over a statistically significant population. It also substantiates whether the drug could be safely consumed by human beings or not. In most cases, the volunteers are paid by these pharma companies running the clinical trial for the drug being tested. In this context, it is of important to confirm whether the drug has been actually consumed by the volunteer or not. In some cases, volunteer may not consume the drug for various reasons thereby reducing the effectiveness and authenticity of the clinical study.

US 9047746 B1 relates to electronic systems and methods for monitoring medication compliance. US 2014009262 relates to a target authentication device that includes an electrode to detect an electrical signal associated with a user of the device. The electrical signal represents an authentication code for the device. There is a need to overcome the disadvantages and limitations of the existing systems.

### Summary of the Disclosure

In accordance with the invention, there is provided: a method of monitoring drug adherence in a clinical trial, as recited by claim 1; a monitoring device for monitoring drug adherence in a clinical trial, as recited by claim 4; and a non-transitory computer readable storage medium, as recited by claim 9.

One or more shortcomings of the prior art are overcome and additional advantages are provided through the present disclosure. Additional features and advantages are realized through the techniques of the present disclosure. Other embodiments and aspects of the disclosure are described in detail herein and are considered a part of the claimed disclosure.

Accordingly, the present disclosure relates to a method of monitoring drug adherence in a clinical trial, said method comprising: receiving, by a monitoring device, one or more first signals indicative of the physiological information associated with a user upon consuming a medication comprising the drug; determining, by the monitoring device, authenticity of the user consuming the medication based on comparison of physiological information associated with the one or more first signals with previously stored physiological information associated with a plurality of concerned users; detecting, by the monitoring device, valid proximity of the monitoring device to the medication consumed by the authenticated user based on signal strength associated with one or more second signals received from the medication; determining, by the monitoring device, successful passing of the medication within gastrointestinal (GI) tract of the user based on one or more biometric signatures generated by the medication during the passing within the GI tract of the authenticated user; and reporting, by the monitoring device, successful drug adherence of the user based on determination of valid proximity of the monitoring device with the medication and successful passing of the medication within the GI tract of the authenticated user.

The step of determining the authenticity of the user may also be referred to as authenticating the user. A user who is determined to be authenticated may be referred to herein as an "authenticated user".

The medication may further comprise a transmitter or more preferably a transceiver. In an embodiment, the step of determining, by the monitoring device, valid proximity of the monitoring device to the medication consumed by the authenticated user is based on a signal strength of one or more second signals emitted from the transmitter (or transceiver).

Further, the present disclosure relates to a monitoring device for monitoring drug adherence in a clinical trial, said system comprising: at least one first physiological sensor capable of receiving one or more first signals indicative of the physiological information associated with a user; a processor coupled with the at least one first physiological sensor; and a memory disposed in communication with the processor for storing processor-executable instructions, the instructions comprising instructions to: receive the one or more first signals upon consuming a medication comprising the drug; determine authenticity of the user consuming the medication based on comparison of physiological information, associated with the one or more first signals, with previously stored physiological information associated with a plurality of concerned users; detect valid proximity of the monitoring device to the medication consumed by the authenticated user based on signal strength associated with one or more second signals received from the medication; determine successful passing of the medication within gastrointestinal (GI) tract of the user based on one or more biometric signatures generated by the medication during the passing within the GI tract of the authenticated user; and report successful drug adherence of the user based on determination of valid proximity of the monitoring device with the medication and successful passing of the medication within the GI tract of the authenticated user.

Further, the present disclosure relates to a method of monitoring drug adherence in a clinical trial. The method comprising the step of receiving one or more first signals indicative of physiological information associated with a user upon consuming a medication comprising the drug. The method further comprising the step of determining authenticity of the user consuming the medication based on comparison of the physiological information associated with the one or more first signals with previously stored physiological information associated with a plurality of concerned users. Upon determining authenticity of the user, valid proximity of the monitoring device to the medication consumed by the authenticated user is determined based on signal strength associated with one or more second signals received from the medication. Upon determining valid proximity, successful passing of the medication within gastrointestinal (GI) tract of the user is determined based on one or more biometric signatures generated by the medication during the passing within the GI tract of the authenticated user. Based on determination of valid proximity of the monitoring device with the medication and successful passing of the medication within the GI tract of the authenticated user, successful drug adherence of the user is reported by the monitoring device.

Further, the present disclosure relates to a system for monitoring drug adherence in a clinical trial. The system comprises at least one first physiological sensor capable of receiving one or more first signals indicative of physiological information associated with a user and a processor coupled with the at least one first physiological sensor. The system further comprises a memory communicatively coupled with the processor, wherein the memory stores processor-executable instructions, which, on execution, cause the processor to receive one or more first signals upon consuming a medication comprising the drug and determine authenticity of the user consuming the medication. The processor is configured to determine the authenticity of the user based on comparison of the physiological information, associated with the one or more first signals, with previously stored physiological information associated with a plurality of concerned users. Upon determining authenticity of the user, the processor is configured to detect valid proximity of the monitoring device to the medication consumed by the authenticated user based on signal strength associated with one or more second signals received from the medication. The processor is further configured to determine successful passing of the medication within gastrointestinal (GI) tract of the user based on one or more biometric signatures generated by the medication during the passing within the GI tract of the authenticated user. Based on determination of valid proximity of the monitoring device with the medication and successful passing of the medication within the GI tract of the authenticated user, the processor is configured to report successful drug adherence of the user.

Furthermore, the present disclosure relates to a non-transitory computer readable medium including instructions stored thereon that when processed by at least one processor cause a system to perform the act of receiving one or more first signals indicative of physiological information associated with the user upon consuming a medication comprising the drug. Further, the instructions cause the processor to determine authenticity of the user consuming the medication based on comparison of the physiological information, associated with the one or more first signals, with previously stored physiological information associated with a plurality of concerned users. The processor is further configured to detect valid proximity of the monitoring device to the medication consumed by the authenticated user based on signal strength associated with one or more second signals received from the medication. Furthermore, the instructions cause the processor to determine successful passing of the medication within gastrointestinal (GI) tract of the user based on one or more biometric signatures generated by the medication during the passing within the GI tract of the authenticated user. The processor is configured to report successful drug adherence of the user based on determination of valid proximity of the monitoring device with the medication and successful passing of the medication within the GI tract of the authenticated user.

Further aspects, embodiments, and features will become apparent by reference to the drawings and the following detailed description.

### Brief description of the drawings

The accompanying drawings, which are incorporated in and constitute a part of this disclosure, illustrate exemplary embodiments and, together with the description, serve to explain the disclosed embodiments. In the figures, the left-most digit(s) of a reference number identifies the figure in which the reference number first appears. The same numbers are used throughout the figures to reference like features and components. Some embodiments of system and/or methods in accordance with embodiments of the present subject matter are now described, by way of example only, and with reference to the accompanying figures, in which:
**Figure 1** illustrates an architecture diagram of an exemplary system for monitoring drug adherence in a clinical trial in accordance with some embodiments of the present disclosure;
**Figure 2** illustrates an exemplary block diagram of a monitoring device of **Figure 1** in accordance with some embodiments of the present disclosure;
**Figure 3** illustrates a flowchart of an exemplary method of monitoring drug adherence in a clinical trial in accordance with some embodiments of the present disclosure; and
**Figure 4** is a block diagram of an exemplary computer system for implementing embodiments consistent with the present disclosure.

It should be appreciated by those skilled in the art that any block diagrams herein represent conceptual views of illustrative systems embodying the principles of the present subject matter. Similarly, it will be appreciated that any flow charts, flow diagrams, state transition diagrams, pseudo code, and the like represent various processes which may be substantially represented in computer readable medium and executed by a computer or processor, whether or not such computer or processor is explicitly shown.

### Detailed description

In the present document, the word "exemplary" is used herein to mean "serving as an example, instance, or illustration." Any embodiment or implementation of the present subject matter described herein as "exemplary" is not necessarily to be construed as preferred or advantageous over other embodiments.

While the disclosure is susceptible to various modifications and alternative forms, specific embodiment thereof has been shown by way of example in the drawings and will be described in detail below. It should be understood, however that it is not intended to limit the disclosure to the particular forms disclosed, but on the contrary, the disclosure is to cover all modifications, equivalents, and alternative falling within the scope of the appended claims.

The terms "comprises", "comprising", or any other variations thereof, are intended to cover a non-exclusive inclusion, such that a setup, device or method that comprises a list of components or steps does not include only those components or steps but may include other components or steps not expressly listed or inherent to such setup or device or method. In other words, one or more elements in a system or apparatus proceeded by "comprises... a" does not, without more constraints, preclude the existence of other elements or additional elements in the system or apparatus.

The present disclosure relates to a monitoring device and a method of monitoring drug adherence in a clinical trial. In one embodiment, the monitoring device determines that only an authenticated user has consumed the medication comprising the drug. Further, the monitoring device determines that the authenticated user who has consumed the drug is associated with the corresponding monitoring device and furthermore determines that the drug has successfully passed through gastrointestinal (GI) tract of the authenticated user based on analysis of one or more biometric signatures of the GI tract. Based on the determination of valid proximity and successful passing of the medication within the GI tract of the authenticated user, the monitoring device reports that the drug adherence by the user is successful. Thus, the disclosed method and the monitoring device determine that only authenticated user has consumed the medication and not just if the medication has been taken by any person.

In the following detailed description of the embodiments of the disclosure, reference is made to the accompanying drawings that form a part hereof, and in which are shown by way of illustration specific embodiments in which the disclosure may be practiced. These embodiments are described in sufficient detail to enable those skilled in the art to practice the disclosure, and it is to be understood that other embodiments may be utilized and that changes may be made without departing from the scope of the appended claims. The following description is, therefore, not to be taken in a limiting sense.

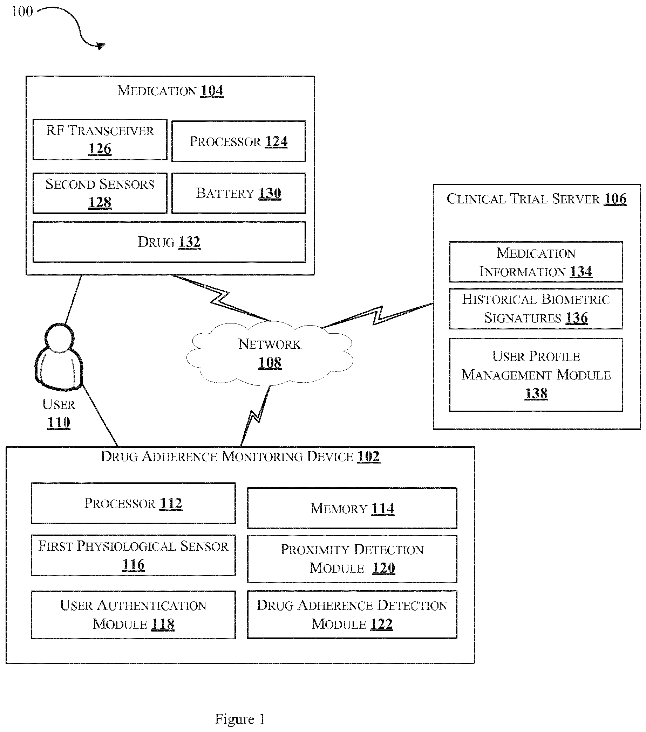

**Figure 1** illustrates an architecture diagram of an exemplary system for monitoring drug adherence in a clinical trial in accordance with some embodiments of the present disclosure.

As shown in **Figure 1****,** the exemplary system **100** comprises one or more components configured for monitoring drug adherence in a clinical trial. In one embodiment, the exemplary system **100** comprises a drug adherence monitoring device (hereinafter referred to as monitoring device) **102,** medication **104** comprising the drug and a clinical trial server **106** connected via a communication network **108.** The monitoring device **102** is configured to determine drug adherence by a user **110** based on information received from the medication **104.** In one embodiment, the monitoring device **102** comprises at least a processor **112,** a memory **114,** at least one first physiological sensor **116,** a user authentication module **118,** a proximity detection module **120** and a drug adherence detection module **122** to determine the drug adherence by the user **110.** The monitoring device **102** communicates with the medication **104** and receives information related to the drug from the medication **104** to determine the drug adherence by the user **110.**

In one implementation, the monitoring device **102** communicates with the medication **104** using wireless technology such as Bluetooth for example. In another implementation, the monitoring device **102** communicates with the medication **104** via the communication network **108.** The medication **104** may be for example a swallowable/ingestible pill or tablet, capable of being consumed by the user **110** undergoing the clinical trial. The medication **104** comprises one or more components made of bio-friendly material that can be easily adaptable by the body of the user **110.** In one embodiment, the medication **104** comprises the one or more components including at least a processor **124** and a RF transceiver **126** coupled with the processor **124.** The medication **104** further comprises one or more second sensors **128,** a battery **130** and drug **132.** The one or more second sensors **128** may be for example, an accelerometer, a pH and/or a temperature sensor and so on capable of collecting information related to acceleration of the medication **104** within gastrointestinal (GI) tract of the user **110,** pH level and temperature of the GI tract of the user **110** when the drug **132** is being absorbed by the GI tract of the user **110.**

Upon collecting the related information by the one or more sensors **128,** the RF transceiver **126** of the medication **104** transmits the collected information to the monitoring device **102.** The battery **130** is capable of providing the necessary power supply to enable the transmission of the collected information to the monitoring device **102.** In one example, the battery **130** is configured to last around 6 to 8 hours. In another example, the battery **130** is configured with an energy harvesting mechanism comprising bimetallic electrodes mounted on the surface of the medication **104** to generate the necessary power supply based on the fluid of the GI tract of the user **110.** The monitoring device **102** determines drug adherence based on the received information and transmits the drug adherence results to the clinical trial server **106.**

In one embodiment, the clinical trial server **106** may be configured by an interested company or organization to conduct the clinical trials and evaluate the results to determine the drug effectiveness. The clinical trial server **106** may also be configured to track information **134** related to the medication **104** including medication identification (ID), dosage information of the medication **104** and so on. The clinical trial server **106** is further configured to store historical biometric signatures **136** of one or more users previously recorded during clinical trials conducted in the past. The clinical trial server **106** also comprises a user profile management module **138** configured to update one or more user profiles with corresponding biometric signatures and drug adherence results provided by the monitoring device **102.**

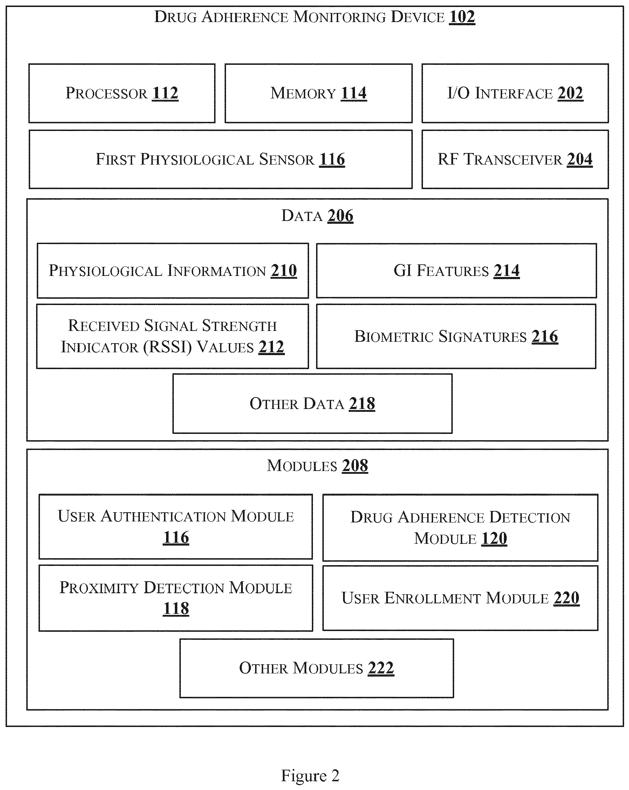

The monitoring device **102** may be a typical monitoring device as illustrated in **Figure 2****.** In one example, the monitoring device **102** may be one of a wearable device, a hand held device and an embedded device. The monitoring device **102** comprises the processor **112,** the memory **114,** the first physiological sensor **116,** I/O interface **202** and an RF transceiver **204.** The I/O interface **202** is coupled with the processor **112** and an I/O device. The I/O device is configured to receive inputs via the I/O interface **202** and transmit outputs for displaying in the I/O device via the I/O interface **202.** The first physiological sensor may be for example, an Electrocardiogram (ECG) sensor, capable of obtaining signals indicative of the heart rate. The RF transceiver **204** is capable of receiving and transmitting information in one or more signals representing the said information.

The monitoring device **102** further comprises data **206** and modules **208.** In one implementation, the data **206** and the modules **208** may be stored within the memory **114.** In one example, the data **206** may include physiological information **210,** Received Signal Strength Indicator (RSSI) **212,** gastrointestinal (GI) features **214,** biometric signatures **216** and other data **218.** In one embodiment, the data **206** may be stored in the memory **114** in the form of various data structures. Additionally, the aforementioned data can be organized using data models, such as relational or hierarchical data models. The other data **218** may be also referred to as reference repository for storing recommended implementation approaches as reference data. The other data **218** may also store data, including temporary data and temporary files, generated by the modules **208** for performing the various functions of the monitoring device **102.**

The modules **208** may include, for example, the user authentication module **116,** the proximity detection module **118,** the drug adherence detection module **120** and a user enrollment module **220.** The modules **208** may also comprise other modules **222** to perform various miscellaneous functionalities of the monitoring device **102.** It will be appreciated that such aforementioned modules may be represented as a single module or a combination of different modules. The modules **208** may be implemented in the form of software, hardware and/or firmware.

In operation, the medication **104** is activated and consumed by the user **110.** In one embodiment, the user **110** consuming the medication **104** is a registered user enrolled with the clinical trial server **106** associated with the interested company or organization. In one implementation, the user enrollment module **220** is configured to register one or more concerned users with the clinical trial server **106** by creating one or more corresponding user profiles and storing the one or more user profiles in the clinical trial server **106.** For this purpose, the one or more concerned users are made to consume a dummy medication without any drug **130.** The first physiological sensor **116,** for example, electrocardiogram (ECG) sensor, is configured to obtain one or more test physiological signals indicative of physiological information i.e., ECG signature, associated with the one or more users. The term "physiological information" is preferably used herein to include data derivable from monitoring physiological processes of a concerned user in the clinical trial. The physiological information may include parameters associated with a concerned user's vital signs. The physiological information may include heart rate and/or pulse oximetry and/or respiratory rate and/or blood pressure.

Based on the physiological information, the drug adherence detection module **120** is configured to generate one or more test biometric signatures, for example GI signatures, associated with the GI tract of the one or more concerned users and determine a test probability value indicating possibility of passing or non-passing of the dummy medication within the GI tract of the one or more concerned users. Upon determination, the user enrollment module **220** updates the one or more user profiles stored in the clinical trial server **106** with corresponding historical biometric signatures **136** i.e., ECG signatures and GI signatures. Further, the user enrollment module **220** updates the one or more user profiles with the corresponding test probability value determined corresponding to the one or more concerned users. The term "biometric signatures" is preferably used herein to refer to patterns derived from physiological information and/or gastrointestinal parameters of the one or more concerned users, which may change in response to successful passing of the drug. In this way, a biometric signature associated with successful passing of a drug through the GI tract may be different to a biometric signature associated with unsuccessful passing of a drug through the GI tract. The physiological information and/or the GI parameters may be continually monitored during the trial to detect a change in biometric signature.

During the clinical trial, the medication **104** is activated before consumption by the user **110** and drug adherence is determined by the monitoring device **102** based on the activity of the medication **104.** In one embodiment, the medication **104** is automatically activated and powered ON by triggering an in-built activation switch coupled with the battery **130** of the medication **104.** In another embodiment, the medication is manually activated by the user **110** by applying a predetermined pressure on the battery **130.** Upon activation, the medication **104** is consumed by the user **110** and the monitoring device **102** determines the drug adherence of the user **110** based on the information received from the medication **104** consumed by the user **110.**

In one embodiment, the monitoring device **102** is configured to determine authenticity of the user **110** consumed the medication **104.** In other words, the monitoring device **102** determines as to whether the user **110** consumed the medication **104** is an authenticated user or not. In one implementation, the user authentication module **118** determines the authenticity of the user **110** associated with the monitoring device **102.** The user authentication module **118** receives one or more first signals indicative of physiological information associated with the user **110** via the first physiological sensor **116.** In one example, the physiological information may be ECG information obtained via the first physiological sensor, for example an ECG sensor and the ECG information may include time and frequency domain features. The user authentication module **118** receives the physiological information and compares the received physiological information with previously stored physiological information associated with one or more concerned users enrolled with the clinical trial server **106.** Based on the comparison, the user authentication module **118** determines that the user **110** who has consumed the medication **104** is a valid user if there is a match in the physiological information. In another implementation, the user authentication module **118** may determine authenticity of the user **110** using any known ECG-based authentication techniques.

If the user authentication module **118** determines that the user **110** is not a valid user, then the monitoring device **102** generates an alarm signal and sends a report to the clinical trial server **106** indicating the unauthenticated consumption of the medication **104.** In such cases, the user enrollment module **220** registers the user **110** with the clinical trial server 106 and the user authentication module **118** resumes the authentication. Upon determining the user **110** as valid and authenticated user, the monitoring device **102** determines proximity of the medication **104** to the monitoring device **102** to determine that the medication **104** is being consumed by the same user who has been authenticated by the monitoring device **102.**

In one embodiment, the proximity detection module **118** is configured to determine proximity of the medication **104** to the user **110** based on information associated with the one or more second signals transmitted by the medication **104** via the RF transceiver **126.** In one implementation, the proximity detection module **118** receives the one or more second signals from the one or more second sensors **128** of the medication **104** via the RF transceiver **204.** The proximity detection module **118** determines the strength of the one or more second signals for example, Received Signal Strength Indicator (RSSI) values of the one or more second signals and compares with a predetermined threshold value. Based on the comparison, the proximity detection module **118** determines the acceptable or valid proximity of the medication **104** for example, 30 to 60 cms, ensuring that the medication **104** is consumed by the same user **110** who has been authenticated by the monitoring device **102.**

In another embodiment, the proximity detection module **118** determines the proximity of the medication **104** to the monitoring device **102** using Near Field Communication (NFC) tech for example. The proximity detection module **118** receives the one or more second signals from the one or more second sensors **128** of the medication **104** when the medication **104** is tapped onto the monitoring device **102** before consumption of the medication **104.** The proximity detection module **118** compares the strength of the one or more second signals with the predetermined threshold value and determines valid proximity of the medication **104** based on the comparison. If the proximity detection module **118** determines unacceptable or invalid proximity of the medication **104** to the monitoring device **102,** then the monitoring device **102** generates an alarm signal indicating the invalid proximity and transmits a corresponding report to the clinical trial server **106** for further operations. Upon determining valid proximity of the medication **104,** the monitoring device **102** determines the drug adherence of the user **110.**

In one embodiment, the drug adherence detection module (hereinafter referred to as DAD module) **120** detect the passage of the medication **104** within the GI tract of the user **110.** The DAD module **120** derives one or more physiological information associated with the one or more second signals received from the medication **104** during the passing within the GI tract of the user **110.** In one example, the DAD module **120** derives the one or more physiological information including acceleration, pH and temperature information associated with the medication **104** when the medication **104** is passing through the GI tract of the user **110.** Based on the derived information, the DAD module **120** determines one or more features associated with the GI tract, for example, a long term feature, a short term feature and peristaltic feature. Long term features, for example, relate to mean and variance of the one or more signals for a time window of 5 to 10 seconds. Short term features are mean and variance of the one or more signals for a time window of for example, 1 to 2 seconds. Peristaltic features is defined as histogram bin count of real and imaginary frequency components of the accelerometer, pH and temperature signatures taken over a time window of for example, 10 to 20 seconds. Based on the one or more features thus determined, the DAD module **120** generates one or more biometric signatures associated with the GI tract and determine probability of passing of the medication **104** within the GI tract based on the one or more biometric signatures thus generated.

In one embodiment, the DAD module **120** compares the one or more biometric signatures thus generated with previously stored historic biometric signatures **136** of the clinical trial server **106.** Based on the comparison, the DAD module **120** determines the probability value corresponding to the one or more matching biometric signatures and compares the probability value with a predetermined threshold probability value, for example 0.6. In one implementation, the predetermined threshold probability value is determined through empirical methods. If the DAD module **120** determines that the probability value exceeds the predetermined threshold probability value, then the DAD module **120** determines that the medication **104** has passed through the GI tract of the user **110.** Otherwise, the DAD module **120** generates an alarm signal indicating the non-passing of the medication **104** within the GI tract of the user **110** and transmits a corresponding report to the clinical trial server **106** for further operations.

Based on successful authentication of the user **110,** valid proximity of the medication **104** to the monitoring device **102** and successful passing of the medication **104** within the GI tract of the user **110,** the DAD module **120** generates a report indicating successful drug adherence by the user **110** and transmits the report to the clinical trial server **106.** In one example, the generated report comprises information associated with the medication 104, one or more biometric signatures determined by the DAD module, test probability value and success/failure status of the drug adherence. On receiving the report, the user profile management module **138** of the clinical trial server **106** updates the user profile of the user **110** with corresponding probability value and derived features for future use. Thus, the system **100** enables monitoring of drug adherence by user identifying any deviation instances where users have not taken the drug and where unauthenticated users have taken the drug and still further whether the drug has successfully passed within the body of the user.

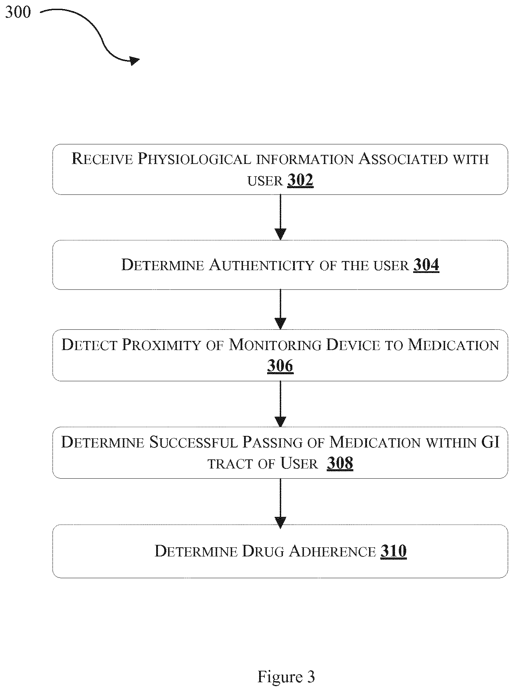

**Figure 3** illustrates a flowchart of a method of monitoring drug adherence in a clinical trial in accordance with some embodiments of the present disclosure.

As illustrated in **Figure 3****,** the method **300** comprises one or more blocks implemented by the processor **112** for monitoring drug adherence in a clinical trial. The method **300** may be described in the general context of computer executable instructions. Generally, computer executable instructions can include routines, programs, objects, components, data structures, procedures, modules, and functions, which perform particular functions or implement particular abstract data types.

The order in which the method **300** is described is not intended to be construed as a limitation, and any number of the described method blocks can be combined in any order to implement the method **300.** Additionally, individual blocks may be deleted from the method **300** without departing from the scope of the subject matter of the appended claims. Furthermore, the method **300** can be implemented in any suitable hardware, software, firmware, or combination thereof.

At block **302,** receive physiological information associated with the user. In one embodiment, the user authentication module **118** receives one or more first signals indicative of physiological information associated with the user **110** via the first physiological sensor **116.** In one example, the physiological information may be ECG information obtained via the first physiological sensor, for example an ECG sensor.

At block **304,** determine authenticity of the user. In one embodiment, the user authentication module **118** determines the authenticity of the user **110** associated with the monitoring device **102.** The user authentication module **118** receives the one or more first signals indicative of physiological information associated with the user **110** via the first physiological sensor **116** and compares the received physiological information with previously stored physiological information associated with one or more concerned users enrolled with the clinical trial server **106.** Based on the comparison, the user authentication module **118** determines that the user **110** who has consumed the medication **104** is a valid user if there is a match in the physiological information. If the user authentication module **118** determines that the user **110** is not a valid user, then the monitoring device **102** generates an alarm signal and sends a report to the clinical trial server **106** indicating the unauthenticated consumption of the medication **104.** Upon determining the user **110** as valid and authenticated user, the monitoring device **102** determines proximity of the medication **104** to the monitoring device **102** to determine that the medication **104** is being consumed by the same user who has been authenticated by the monitoring device **102.**

At block **306,** detect proximity of the monitoring device to the medication. In one embodiment, the proximity detection module **118** is configured to determine proximity of the medication **104** to the user **110** based on information associated with the one or more second signals transmitted by the medication **104** via the RF transceiver **126.** In one implementation, the proximity detection module **118** receives the one or more second signals from the one or more second sensors **128** of the medication **104** via the RF transceiver **204.** The proximity detection module **118** determines the strength of the one or more second signals for example, Received Signal Strength Indicator (RSSI) values of the one or more second signals and compares with a predetermined threshold value. Based on the comparison, the proximity detection module **118** determines the acceptable or valid proximity of the medication **104** ensuring that the medication **104** is consumed by the same user **110** who has been authenticated by the monitoring device **102.**

In another embodiment, the proximity detection module **118** determines the proximity of the medication **104** to the monitoring device **102** using Near Field Communication (NFC) tech for example. The proximity detection module **118** receives the one or more second signals from the one or more second sensors **128** of the medication **104** when the medication **104** is tapped onto the monitoring device **102** before consumption of the medication **104.** The proximity detection module **118** compares the strength of the one or more second signals with the predetermined threshold value and determines valid proximity of the medication **104** based on the comparison. If the proximity detection module **118** determines unacceptable or invalid proximity of the medication **104** to the monitoring device **102,** then the monitoring device **102** generates an alarm signal indicating the invalid proximity and transmits a corresponding report to the clinical trial server **106** for further operations. Upon determining valid proximity of the medication **104,** the monitoring device **102** determines the drug adherence of the user **110.**

At block **308,** determine successful passing of the medication within the GI tract of the user. In one embodiment, the DAD module **120** detects the passage of the medication **104** within the GI tract of the user **110.** The DAD module **120** derives one or more physiological information associated with the one or more second signals received from the medication **104** during the passing within the GI tract of the user **110.** In one example, the DAD module **120** derives the one or more physiological information including acceleration, pH and temperature information associated with the medication **104** when the medication **104** is passing through the GI tract of the user **110.** Based on the derived information, the DAD module **120** determines one or more features associated with the GI tract, for example, a long term feature, a short term feature and peristaltic feature. Based on the one or more features thus determined, the DAD module **120** generates one or more biometric signatures associated with the GI tract and determine probability of passing of the medication 104 within the GI tract based on the one or more biometric signatures thus generated.

In one embodiment, the DAD module **120** compares the one or more biometric signatures thus generated with previously stored historic biometric signatures **136** of the clinical trial server **106.** Based on the comparison, the DAD module **120** determines the probability value corresponding to the one or more matching biometric signatures and compares the probability value with a predetermined threshold probability value. If the DAD module **120** determines that the probability value exceeds the predetermined threshold probability value, then the DAD module **120** determines that the medication **104** has passed through the GI tract of the user **110.** Otherwise, the DAD module **120** generates an alarm signal indicating the non-passing of the medication **104** within the GI tract of the user **110** and transmits a corresponding report to the clinical trial server **106** for further operations.

At block **310,** determine drug adherence. In one embodiment, the monitoring device determines successful drug adherence by the user based on successful authentication of the user **110,** valid proximity of the medication **104** to the monitoring device **102** and successful passing of the medication **104** within the GI tract of the user **110.** In one implementation, the DAD module **120** generates a report indicating successful drug adherence by the user **110** and transmits the report to the clinical trial server **106.** On receiving the report, the user profile management module **138** of the clinical trial server **106** updates the user profile of the user **110** with corresponding probability value and derived features for future use.

Thus, the system **100** enables monitoring of drug adherence by user identifying any deviation instances where users have not taken the drug and where unauthenticated users have taken the drug and still further whether the drug has successfully passed within the body of the user.

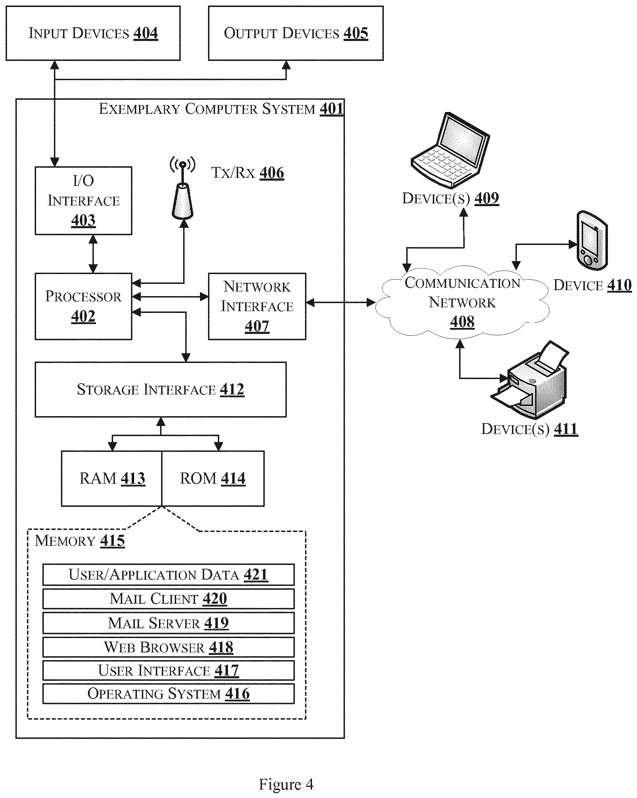

**Figure 4** is a block diagram of an exemplary computer system for implementing embodiments consistent with the present disclosure.

Variations of computer system **401** may be used for implementing all the computing systems that may be utilized to implement the features of the present disclosure. Computer system **401** may comprise a central processing unit ("CPU" or "processor") **402.** Processor **402** may comprise at least one data processor for executing program components for executing user- or system-generated requests. The processor may include specialized processing units such as integrated system (bus) controllers, memory management control units, floating point units, graphics processing units, digital signal processing units, etc. The processor **402** may include a microprocessor, such as AMD Athlon, Duron or Opteron, ARM's application, embedded or secure processors, IBM PowerPC, Intel's Core, Itanium, Xeon, Celeron or other line of processors, etc. The processor **402** may be implemented using mainframe, distributed processor, multi-core, parallel, grid, or other architectures. Some embodiments may utilize embedded technologies like application-specific integrated circuits (ASICs), digital signal processors (DSPs), Field Programmable Gate Arrays (FPGAs), etc.

Processor **402** may be disposed in communication with one or more input/output (I/O) devices via I/O interface **403.** The I/O interface **403** may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.n /b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface **403,** the computer system **401** may communicate with one or more I/O devices. For example, the input device **404** may be an antenna, keyboard, mouse, joystick, (infrared) remote control, camera, card reader, fax machine, dongle, biometric reader, microphone, touch screen, touchpad, trackball, sensor (e.g., accelerometer, light sensor, GPS, gyroscope, proximity sensor, or the like), stylus, scanner, storage device, transceiver, video device/source, visors, etc. Output device **405** may be a printer, fax machine, video display (e.g., cathode ray tube (CRT), liquid crystal display (LCD), light-emitting diode (LED), plasma, or the like), audio speaker, etc. In some embodiments, a transceiver **406** may be disposed in connection with the processor **402.** The transceiver may facilitate various types of wireless transmission or reception. For example, the transceiver may include an antenna operatively connected to a transceiver chip (e.g., Texas Instruments WiLink WL1283, Broadcom BCM4750IUB8, Infineon Technologies X-Gold 618-PMB9800, or the like), providing IEEE 802.11a/b/g/n, Bluetooth, FM, global positioning system (GPS), 2G/3G HSDPA/HSUPA communications, etc.

In some embodiments, the processor **402** may be disposed in communication with a communication network **408** via a network interface **407.** The network interface **407** may communicate with the communication network **408.** The network interface **407** may employ connection protocols including, without limitation, direct connect, Ethernet (e.g., twisted pair 10/40/400 Base T), transmission control protocol/internet protocol (TCP/IP), token ring, IEEE 802.11a/b/g/n/x, etc. The communication network **408** may include, without limitation, a direct interconnection, local area network (LAN), wide area network (WAN), wireless network (e.g., using Wireless Application Protocol), the Internet, etc. Using the network interface **407** and the communication network **408,** the computer system **401** may communicate with devices **409, 410,** and **411.** These devices may include, without limitation, personal computer(s), server(s), fax machines, printers, scanners, various mobile devices such as cellular telephones, smartphones (e.g., Apple iPhone, Blackberry, Android-based phones, etc.), tablet computers, eBook readers (Amazon Kindle, Nook, etc.), laptop computers, notebooks, gaming consoles (Microsoft Xbox, Nintendo DS, Sony PlayStation, etc.), or the like. In some embodiments, the computer system **401** may itself embody one or more of these devices.

In some embodiments, the processor **402** may be disposed in communication with one or more memory devices (e.g., RAM **413,** ROM **414,** etc.) via a storage interface **412.** The storage interface may connect to memory devices including, without limitation, memory drives, removable disc drives, etc., employing connection protocols such as serial advanced technology attachment (SATA), integrated drive electronics (IDE), IEEE-1394, universal serial bus (USB), fiber channel, small computer systems interface (SCSI), etc. The memory drives may further include a drum, magnetic disc drive, magneto-optical drive, optical drive, redundant array of independent discs (RAID), solid-state memory devices, solid-state drives, etc.

The memory **415** may store a collection of program or database components, including, without limitation, an operating system **416,** user interface application **517,** web browser **418,** mail server **419,** mail client **420,** user/application data **421** (e.g., any data variables or data records discussed in this disclosure), etc. The operating system **416** may facilitate resource management and operation of the computer system **401.** Examples of operating systems include, without limitation, Apple Macintosh OS X, UNIX, Unix-like system distributions (e.g., Berkeley Software Distribution (BSD), FreeBSD, NetBSD, OpenBSD, etc.), Linux distributions (e.g., Red Hat, Ubuntu, Kubuntu, etc.), IBM OS/2, Microsoft Windows (XP, Vista/7/8, etc.), Apple iOS, Google Android, Blackberry OS, or the like. User interface **417** may facilitate display, execution, interaction, manipulation, or operation of program components through textual or graphical facilities. For example, user interfaces may provide computer interaction interface elements on a display system operatively connected to the computer system **401,** such as cursors, icons, check boxes, menus, scrollers, windows, widgets, etc. Graphical user interfaces (GUIs) may be employed, including, without limitation, Apple Macintosh operating systems' Aqua, IBM OS/2, Microsoft Windows (e.g., Aero, Metro, etc.), Unix X-Windows, web interface libraries (e.g., ActiveX, Java, Javascript, AJAX, HTML, Adobe Flash, etc.), or the like.

In some embodiments, the computer system **401** may implement a web browser **418** stored program component. The web browser may be a hypertext viewing application, such as Microsoft Internet Explorer, Google Chrome, Mozilla Firefox, Apple Safari, etc. Secure web browsing may be provided using HTTPS (secure hypertext transport protocol), secure sockets layer (SSL), Transport Layer Security (TLS), etc. Web browsers may utilize facilities such as AJAX, DHTML, Adobe Flash, JavaScript, Java, application programming interfaces (APIs), etc. In some embodiments, the computer system **401** may implement a mail server **419** stored program component. The mail server may be an Internet mail server such as Microsoft Exchange, or the like. The mail server may utilize facilities such as ASP, ActiveX, ANSI C++/C#, Microsoft .NET, CGI scripts, Java, JavaScript, PERL, PHP, Python, WebObjects, etc. The mail server may utilize communication protocols such as internet message access protocol (IMAP), messaging application programming interface (MAPI), Microsoft Exchange, post office protocol (POP), simple mail transfer protocol (SMTP), or the like. In some embodiments, the computer system **401** may implement a mail client **420** stored program component. The mail client may be a mail viewing application, such as Apple Mail, Microsoft Entourage, Microsoft Outlook, Mozilla Thunderbird, etc.

In some embodiments, computer system **401** may store user/application data **421,** such as the data, variables, records, etc. as described in this disclosure. Such databases may be implemented as fault-tolerant, relational, scalable, secure databases such as Oracle or Sybase. Alternatively, such databases may be implemented using standardized data structures, such as an array, hash, linked list, struct, structured text file (e.g., XML), table, or as object-oriented databases (e.g., using ObjectStore, Poet, Zope, etc.). Such databases may be consolidated or distributed, sometimes among the various computer systems discussed above in this disclosure. It is to be understood that the structure and operation of the any computer or database component may be combined, consolidated, or distributed in any working combination.

As described above, the modules **208,** amongst other things, include routines, programs, objects, components, and data structures, which perform particular tasks or implement particular abstract data types. The modules **208** may also be implemented as, signal processor(s), state machine(s), logic circuitries, and/or any other device or component that manipulate signals based on operational instructions. Further, the modules **208** can be implemented by one or more hardware components, by computer-readable instructions executed by a processing unit, or by a combination thereof.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., are non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A method (300) of monitoring drug adherence in a clinical trial, said method (300) comprising:
receiving (302), by a monitoring device (102), from one or more first physiological sensors (116) coupled with the monitoring device (102), one or more first signals indicative of physiological information associated with a user upon consuming a medication (104) comprising the drug;
determining (304), by the monitoring device (102), authenticity of the user consuming the medication (104) based on comparison of physiological information associated with the one or more first signals with previously stored physiological information associated with a plurality of concerned users;
**characterized in that** the method of monitoring drug adherence in a clinical trial comprises:
detecting (306), by the monitoring device (102), valid proximity of the monitoring device (102) to the medication (104) consumed by the authenticated user based on signal strength associated with one or more second signals received from the medication (104);
determining (308), by the monitoring device (102), successful passing of the medication (104) within a gastrointestinal tract of the authenticated user based on one or more biometric signatures generated by the medication (104) during the passing within the gastrointestinal tract of the authenticated user,
wherein determining (308) successful passing of the medication (104) within the gastrointestinal tract of the authenticated user comprises:
deriving at least one of acceleration, pH, and temperature information of the medication (104) from the one or more second signals during the passing of the medication (104) within the gastrointestinal tract of the authenticated user,
determining one or more features including at least a long term feature, a short term feature, and a peristaltic feature associated with the gastrointestinal tract, based on the derived information,
generating the one or more biometric signatures associated with the gastrointestinal tract based on the determined features,
determining probability of passing of the medication (104) within the gastrointestinal tract of the authenticated user based on the one or more biometric signatures,
comparing the probability of passing with a predetermined threshold probability, and
determining (308) successful passing of the medication (104) within the gastrointestinal tract of the authenticated user based on the comparing; and
reporting (310), by the monitoring device (102), successful drug adherence of the authenticated user based on determination of valid proximity of the monitoring device (102) with the medication (104) and successful passing of the medication (104) within the gastrointestinal tract of the authenticated user.

2. The method (300) as claimed in claim 1, wherein detecting (306) valid proximity of the monitoring device (102) to the medication comprises the steps of:
receiving one or more second signals from one or more second sensors (128) configured in the medication (104);
comparing a Received Signal Strength Indicator value of each of the one or more second signals with a predetermined threshold value; and
determining that the monitoring device (102) is located in a valid proximity to the medication (104) consumed by the authenticated user, based on the comparing with the predetermined threshold value.

3. The method (300) as claimed in any preceding claim, further comprising:
creating one or more user profiles for the one or more concerned users;
obtaining one or more test physiological signals from the monitoring device (102) indicative of physiological information associated with the one or more concerned users upon consumption of a dummy medication (104);
generating one or more test biometric signatures associated with the gastrointestinal tract of the one or more users;
determining a test probability value indicating one of passing and non-passing of the dummy medication (104) within the gastrointestinal tract of the one or more concerned users; and
updating the one or more user profiles with the corresponding physiological information and test probability value corresponding to the one or more concerned users.

4. A monitoring device (102) for monitoring drug adherence in a clinical trial, said device (102) comprising:
at least one first physiological sensor capable of receiving one or more first signals indicative of physiological information associated with a user;
a processor (112) coupled with the at least one first physiological sensor (116); and
a memory (114) disposed in communication with the processor (112) for storing processor-executable instructions, the instructions comprising instructions to:
receive (302) the one or more first signals upon consuming a medication (104) comprising the drug;
determine (304) authenticity of the user consuming the medication (104) based on comparison of physiological information, associated with the one or more first signals, with previously stored physiological information associated with a plurality of concerned users;
**characterized in that** the instructions further comprise instructions to:
detect (306) valid proximity of the monitoring device (102) to the medication (104) consumed by the authenticated user based on signal strength associated with one or more second signals received from the medication (104);
determine (308) successful passing of the medication (104) within a gastrointestinal tract of the user based on one or more biometric signatures generated by the medication (104) during the passing within the gastrointestinal tract of the authenticated user,
wherein the processor (112) is configured to determine successful passing of the medication (104) within the gastrointestinal tract of the authenticated user by performing the steps of:
deriving at least one of acceleration, pH, and temperature information of the medication (104) from the one or more second signals during the passing of the medication (104) within the gastrointestinal tract of the authenticated user,
determining one or more features including at least a long term feature, a short term feature, and a peristaltic feature associated with the gastrointestinal tract, based on the derived information,
generating the one or more biometric signatures associated with the gastrointestinal tract based on the determined features,
determining probability of passing of the medication (104) within the gastrointestinal tract of the authenticated user based on the one or more biometric signatures,
comparing the probability of passing with a predetermined threshold probability, and
determining successful (308) passing of the medication (104) within the gastrointestinal tract of the authenticated user based on the comparing; and
report (310) successful drug adherence of the authenticated user based on determination of valid proximity of the monitoring device (102) with the medication (104) and successful passing of the medication (104) within the gastrointestinal tract of the authenticated user.

5. The monitoring device (102) as claimed in claim 4, wherein the processor (112) is configured to detect (306) valid proximity of the monitoring device (102) to the medication (104) by performing the steps of:
receiving one or more second signals from the one or more second sensors configured in the medication (104);
comparing a Received Signal Strength Indicator value of each of the one or more second signals with a predetermined threshold value; and
determining that the monitoring device (102) is located in a valid proximity to the medication (104) consumed by the authenticated user, based on the comparing with the predefined threshold value.

6. The monitoring device (102) as claimed in claim 4 or 5, wherein the processor (124) is further configured to activate the medication (104) by triggering an activation switch coupled with the medication (104) before consumption of the medication (104) by the user.

7. The monitoring device (102) as claimed in any of claims 4 to 6, the processor (112) is further configured to:
create one or more user profiles for the one or more concerned users;
obtain one or more test signals from the monitoring device (102) indicative of physiological information associated with the one or more concerned users upon consumption of a dummy medication (104);
generate one or more test biometric signatures associated with the gastrointestinal tract of the one or more users;
determine a test probability value indicating one of passing and non-passing of the dummy medication (104) within the gastrointestinal tract of the one or more concerned users; and
update the one or more user profiles with the corresponding physiological information and test probability value corresponding to the one or more concerned users.

8. The monitoring device (102) as claimed in any of claims claim 4 to 7, wherein the monitoring device (102) is configured as one of a wearable device, a hand held device and an embedded device.

9. A non-transitory computer readable medium including instructions stored thereon that when processed by at least one processor (112) cause a system (100) to perform the method (300) of any of claims 1 to 3.

## Patentansprüche

1. Verfahren (300) zum Überwachen der Arzneimitteladhärenz in einer klinischen Studie, wobei das Verfahren (300) Folgendes umfasst:
Empfangen (302) durch eine Überwachungsvorrichtung (102) von einem oder mehreren ersten physiologischen Sensoren (116), die mit der Überwachungsvorrichtung (102) gekoppelt sind, von einem oder mehreren ersten Signalen, die physiologische Informationen anzeigen, die mit einem Benutzer beim Konsumieren eines Medikaments (104) verbunden sind, das das Arzneimittel umfasst;
Bestimmen (304) der Authentizität des Benutzers, der das Medikament (104) konsumiert, durch die Überwachungsvorrichtung (102), basierend auf dem Vergleich von physiologischen Informationen, die mit dem einen oder den mehreren ersten Signalen mit zuvor gespeicherten physiologischen Informationen verbunden sind, die mit einer Vielzahl betroffener Benutzer verbunden sind;
**dadurch gekennzeichnet, dass** das Verfahren des Überwachens der Arzneimitteladhärenz in einer klinischen Studie Folgendes umfasst:
Erfassen (306) von gültiger Nähe der Überwachungsvorrichtung (102) zu dem Medikament (104), das durch den authentifizierten Benutzer konsumiert wird, durch die Überwachungsvorrichtung (102), basierend auf der Signalstärke, die mit einem oder mehreren von dem Medikament (104) empfangenen zweiten Signalen verbunden ist;
Bestimmen (308) des erfolgreichen Passierens des Medikaments (104) innerhalb eines Gastrointestinaltrakts des authentifizierten Benutzers durch die Überwachungsvorrichtung (102), basierend auf einer oder mehreren biometrischen Signaturen, die durch das Medikament (104) während des Passierens innerhalb des Gastrointestinaltrakts des authentifizierten Benutzers erzeugt werden,
wobei das Bestimmen (308) des erfolgreichen Passierens des Medikaments (104) innerhalb des Gastrointestinaltrakts des authentifizierten Benutzers Folgendes umfasst:
Ableiten zumindest einer der Beschleunigungs-, pH- und Temperaturinformationen des Medikaments (104) von dem einen oder den mehreren zweiten Signalen während des Passierens des Medikaments (104) innerhalb des Gastrointestinaltrakts des authentifizierten Benutzers,
Bestimmen eines oder mehrerer Merkmale, einschließlich zumindest eines Langzeitmerkmals, eines Kurzzeitmerkmals und eines peristaltischen Merkmals, die mit dem Gastrointestinaltrakt verbunden sind, basierend auf den abgeleiteten Informationen,
Erzeugen der einen oder der mehreren biometrischen Signaturen, die mit dem Gastrointestinaltrakt verbunden sind, basierend auf den bestimmten Merkmalen,
Bestimmen der Wahrscheinlichkeit des Passierens des Medikaments (104) innerhalb des Gastrointestinaltrakts des authentifizierten Benutzers, basierend auf der einen oder den mehreren biometrischen Signaturen,
Vergleichen der Wahrscheinlichkeit des Passierens mit einer vorbestimmten Schwellenwertwahrscheinlichkeit, und
Bestimmen (308) des erfolgreichen Passierens des Medikaments (104) innerhalb des Gastrointestinaltrakts des authentifizierten Benutzers, basierend auf dem Vergleichen; und
Berichten (310) der erfolgreichen Arzneimitteladhärenz des authentifizierten Benutzers durch die Überwachungsvorrichtung (102), basierend auf der Bestimmung von gültiger Nähe der Überwachungsvorrichtung (102) zu dem Medikament (104) und des erfolgreichen Passierens des Medikaments (104) innerhalb des Gastrointestinaltrakt des authentifizierten Benutzers.

2. Verfahren (300), wie in Anspruch 1 beansprucht, wobei das Erfassen (306) von gültiger Nähe der Überwachungsvorrichtung (102) zu dem Medikament die folgenden Schritte umfasst:
Empfangen eines oder mehrerer zweiter Signale von einem oder mehreren zweiten Sensoren (128), die in dem Medikament (104) konfiguriert sind;
Vergleichen eines Indikatorwerts für die Empfangene Signalstärke jedes der einen oder der mehreren zweiten Signale mit einem vorbestimmten Schwellenwert; und
Bestimmen, dass sich die Überwachungsvorrichtung (102) in einer gültigen Nähe zu dem durch den authentifizierten Benutzer konsumierten Medikament (104) befindet, basierend auf dem Vergleichen mit dem vorbestimmten Schwellenwert.

3. Verfahren (300), wie in einem vorhergehenden Anspruch beansprucht, das ferner Folgendes umfasst:
Erstellen eines oder mehrerer Benutzerprofile für den einen oder die mehreren betroffenen Benutzer;
Erhalten eines oder mehrerer physiologischer Testsignale von der Überwachungsvorrichtung (102), die physiologischen Informationen anzeigen, die mit dem einen oder den mehreren betroffenen Benutzern beim Konsum eines Scheinmedikaments (104) verbunden sind;
Erzeugen einer oder mehrerer biometrischer Testsignaturen, die mit dem Gastrointestinaltrakt des einen oder der mehreren Benutzer verbunden sind;
Bestimmen eines Testwahrscheinlichkeitswertes, der eines von Passieren oder Nichtpassieren des Scheinmedikaments (104) innerhalb des Gastrointestinaltrakts des einen oder der mehreren betroffenen Benutzer anzeigt; und
Aktualisieren des einen oder der mehreren Benutzerprofile mit den entsprechenden physiologischen Informationen und dem Testwahrscheinlichkeitswert, der dem einen oder den mehreren betroffenen Benutzern entspricht.

4. Überwachungsvorrichtung (102) zum Überwachen der Arzneimitteladhärenz in einer klinischen Studie, wobei die Vorrichtung (102) Folgendes umfasst:
zumindest einen ersten physiologischen Sensor, der fähig ist, ein oder mehrere erste Signale zu empfangen, die physiologische Informationen anzeigen, die mit einem Benutzer verbunden sind;
einen Prozessor (112), der mit dem zumindest einen ersten physiologischen Sensor (116) gekoppelt ist; und
einen Speicher (114), der in Kommunikation mit dem Prozessor (112) zum Speichern prozessorausführbarer Anweisungen angeordnet ist, wobei die Anweisungen Anweisungen umfassen zum:
Empfangen (302) des einen oder der mehreren ersten Signale beim Konsumieren eines Medikaments (104), das das Arzneimittel umfasst;
Bestimmen (304) der Authentizität des Benutzers, der das Medikament (104) konsumiert, basierend auf dem Vergleich von physiologischen Informationen, die mit dem einen oder den mehreren ersten Signalen mit zuvor gespeicherten physiologischen Informationen verbunden sind, die mit einer Vielzahl betroffener Benutzer verbunden sind;
**dadurch gekennzeichnet, dass** die Anweisungen ferner Anweisungen umfassen zum:
Erkennen (306) von gültiger Nähe der Überwachungsvorrichtung (102) zu dem Medikament (104), das durch den authentifizierten Benutzer konsumiert wird, basierend auf der
Signalstärke, die mit einem oder mehreren von dem Medikament (104) empfangenen zweiten Signalen verbunden ist;
Bestimmen (308) des erfolgreichen Passierens des Medikaments (104) innerhalb eines Gastrointestinaltrakts des Benutzers, basierend auf einer oder mehreren biometrischen Signaturen, die durch das Medikament (104) während des Passierens innerhalb des Gastrointestinaltrakts des authentifizierten Benutzers erzeugt werden,
wobei der Prozessor (112) konfiguriert ist, um das erfolgreiche Passieren des Medikaments (104) innerhalb des Gastrointestinaltrakts des authentifizierten Benutzers zu bestimmen, indem die folgenden Schritte durchgeführt werden:
Ableiten zumindest einer der Beschleunigungs-, pH- und Temperaturinformationen des Medikaments (104) von dem einen oder den mehreren zweiten Signalen während des Passierens des Medikaments (104) innerhalb des Gastrointestinaltrakts des authentifizierten Benutzers,
Bestimmen eines oder mehrerer Merkmale, einschließlich zumindest eines Langzeitmerkmals, eines Kurzzeitmerkmals und eines peristaltischen Merkmals, die mit dem Gastrointestinaltrakt verbunden sind, basierend auf den abgeleiteten Informationen,
Erzeugen der einen oder der mehreren biometrischen Signaturen, die mit dem Gastrointestinaltrakt verbunden sind, basierend auf den bestimmten Merkmalen,
Bestimmen der Wahrscheinlichkeit des Passierens des Medikaments (104) innerhalb des Gastrointestinaltrakts des authentifizierten Benutzers, basierend auf der einen oder den mehreren biometrischen Signaturen,
Vergleichen der Wahrscheinlichkeit des Passierens mit einer vorbestimmten Schwellenwertwahrscheinlichkeit, und
Bestimmen des erfolgreichen (308) Passierens des Medikaments (104) innerhalb des Gastrointestinaltrakts des authentifizierten Benutzers, basierend auf dem Vergleichen; und
Berichten (310) der erfolgreichen Arzneimitteladhärenz des authentifizierten Benutzers, basierend auf der Bestimmung von gültiger Nähe der Überwachungsvorrichtung (102) zu dem Medikament (104) und erfolgreichen Passierens des
Medikaments (104) innerhalb des Gastrointestinaltrakts des authentifizierten Benutzers.

5. Überwachungsvorrichtung (102), wie in Anspruch 4 beansprucht, wobei der Prozessor (112) konfiguriert ist, um gültige Nähe der Überwachungsvorrichtung (102) zu dem Medikament (104) zu erfassen (306), indem die folgenden Schritte durchgeführt werden:
Empfangen eines oder mehrerer zweiter Signale von dem einen oder den mehreren zweiten Sensoren, die in dem Medikament (104) konfiguriert sind;
Vergleichen eines Indikatorwerts für die Empfangene Signalstärke jedes der einen oder der mehreren zweiten Signale mit einem vorbestimmten Schwellenwert; und
Bestimmen, dass sich die Überwachungsvorrichtung (102) in einer gültigen Nähe zu dem durch den authentifizierten Benutzer konsumierten Medikament (104) befindet, basierend auf dem Vergleichen mit dem vordefinierten Schwellenwert.

6. Überwachungsvorrichtung (102), wie in einem der Ansprüche 4 oder 5 beansprucht, wobei der Prozessor (124) ferner konfiguriert ist, um das Medikament (104) durch Auslösen eines mit dem Medikament (104) gekoppelten Aktivierungsschalters vor dem Konsum des Medikaments (104) durch den Benutzer zu aktivieren.

7. Überwachungsvorrichtung (102), wie in einem der Ansprüche 4 bis 6 beansprucht, wobei der Prozessor (112) ferner konfiguriert ist zum:
Erstellen eines oder mehrerer Benutzerprofile für den einen oder die mehreren betroffenen Benutzer;
Erhalten eines oder mehrerer Testsignale von der Überwachungsvorrichtung (102), die physiologischen Informationen anzeigen, die mit dem einen oder den mehreren betroffenen Benutzern beim Konsum eines Scheinmedikaments (104) verbunden sind;
Erzeugen eines oder mehrerer biometrischer Testsignaturen, die mit dem Gastrointestinaltrakt des einen oder der mehreren Benutzer verbunden sind;
Bestimmen eines Testwahrscheinlichkeitswertes, der eines von Passieren und Nichtpassieren des Scheinmedikaments (104) innerhalb des Gastrointestinaltrakts des einen oder der mehreren betroffenen Benutzer anzeigt; und
Aktualisieren des einen oder der mehreren Benutzerprofile mit den entsprechenden physiologischen Informationen und dem Testwahrscheinlichkeitswert, der dem einen oder den mehreren betroffenen Benutzern entspricht.

8. Überwachungsvorrichtung (102), wie in einem der Ansprüche 4 bis 7 beansprucht, wobei die Überwachungsvorrichtung (102) als eine von einer tragbaren Vorrichtung, einer handgehaltenen Vorrichtung und einer eingebetteten Vorrichtung konfiguriert ist.

9. Nichtflüchtiges computerlesbares Medium, einschließlich darauf gespeicherter Anweisungen, die, wenn sie durch wenigstens einen Prozessor (112) verarbeitet werden, ein System (100) veranlassen, das Verfahren (300) nach einem der Ansprüche 1 bis 3 durchzuführen.

## Revendications

1. Procédé (300) de surveillance de l'adhérence d'un médicament dans un essai clinique, ledit procédé (300) comprenant :
la réception (302), par un dispositif de surveillance (102), en provenance d'un ou plusieurs premiers capteurs physiologiques (116) couplés au dispositif de surveillance (102), d'un ou plusieurs premiers signaux indiquant des informations physiologiques associées à un utilisateur lors de la consommation d'un remède (104) comprenant le médicament ;
la détermination (304), par le dispositif de surveillance (102), de l'authenticité de l'utilisateur consommant le remède (104) sur la base d'une comparaison d'informations physiologiques associées auxdits un ou plusieurs premiers signaux à des informations physiologiques précédemment stockées associées à une pluralité d'utilisateurs concernés ;
**caractérisé en ce que** le procédé de surveillance de l'adhérence d'un médicament dans un essai clinique comprend :
la détection (306), par le dispositif de surveillance (102), d'une proximité valide du dispositif de surveillance (102) avec le remède (104) consommé par l'utilisateur authentifié sur la base de l'intensité du signal associée à un ou plusieurs seconds signaux reçus en provenance du remède (104) ;
la détermination (308), par le dispositif de surveillance (102), du passage réussi du remède (104) dans le tube digestif de l'utilisateur authentifié sur la base d'une ou plusieurs signatures biométriques générées par le remède (104) pendant le passage dans le tube digestif de l'utilisateur authentifié,
ladite détermination (308) du passage réussi du remède (104) dans le tube digestif de l'utilisateur authentifié comprenant :
la dérivation d'au moins l'une des informations d'accélération, de pH et de température du remède (104) provenant desdits un ou plusieurs seconds signaux pendant le passage du remède (104) dans le tube digestif de l'utilisateur authentifié,
la détermination d'une ou plusieurs caractéristiques dont au moins une caractéristique à long terme, une caractéristique à court terme et une caractéristique péristaltique associée au tube digestif, sur la base des informations dérivées,
la génération desdites une ou plusieurs signatures biométriques associées au tube digestif sur la base des caractéristiques déterminées,
la détermination de la probabilité de passage du remède (104) dans le tube digestif de l'utilisateur authentifié sur la base desdites une ou plusieurs signatures biométriques,
la comparaison de la probabilité de passage avec une probabilité seuil préétablie, et
la détermination (308) du passage réussi du remède (104) dans le tube digestif de l'utilisateur authentifié sur la base de la comparaison ; et
le signalement (310), par le dispositif de surveillance (102), d'une adhérence d'un médicament réussie de l'utilisateur authentifié sur la base de la détermination de la proximité valide du dispositif de surveillance (102) avec le remède (104) et du passage réussi du remède (104) dans le tube digestif de l'utilisateur authentifié.

2. Procédé (300) selon la revendication 1, ladite détection (306) de la proximité valide du dispositif de surveillance (102) par rapport au remède comprenant les étapes de :
réception d'un ou plusieurs seconds signaux en provenance d'un ou plusieurs seconds capteurs (128) configurés dans le remède (104) ;
comparaison d'une valeur d'indicateur d'intensité de signal reçu de chacun desdits un ou plusieurs seconds signaux à une valeur seuil préétablie ; et
détermination que le dispositif de surveillance (102) est situé à une proximité valide du remède (104) consommé par l'utilisateur authentifié, sur la base de la comparaison à la valeur seuil préétablie.

3. Procédé (300) selon l'une quelconque des revendications précédentes, comprenant en outre :
la création d'un ou plusieurs profils d'utilisateurs pour lesdits un ou plusieurs utilisateurs concernés ;
l'obtention d'un ou plusieurs signaux physiologiques de test provenant du dispositif de surveillance (102) indiquant des informations physiologiques associées auxdits un ou plusieurs utilisateurs concernés lors de la consommation d'un placebo (104) ;
la génération d'une ou plusieurs signatures biométriques de test associées au tube digestif desdits un ou plusieurs utilisateurs ;
la détermination d'une valeur de probabilité de test indiquant l'un du passage et du non-passage du placebo (104) dans le tube digestif desdits un ou plusieurs utilisateurs concernés ; et
la mise à jour desdits un ou plusieurs profils d'utilisateurs avec les informations physiologiques correspondantes et la valeur de probabilité de test correspondant auxdits un ou plusieurs utilisateurs concernés.

4. Dispositif de surveillance (102) permettant de surveiller l'adhérence d'un médicament dans un essai clinique, ledit dispositif (102) comprenant :
au moins un premier capteur physiologique capable de recevoir un ou plusieurs premiers signaux indiquant des informations physiologiques associées à un utilisateur ;
un processeur (112) couplé audit au moins un premier capteur physiologique (116) ; et
une mémoire (114) disposée en communication avec le processeur (112) pour stocker des instructions exécutables par processeur, les instructions comprenant des instructions pour :
recevoir (302) lesdits un ou plusieurs premiers signaux lors de la consommation d'un remède (104) comprenant le médicament ;
déterminer (304) l'authenticité de l'utilisateur consommant le remède (104) sur la base de la comparaison d'informations physiologiques, associées auxdits un ou plusieurs premiers signaux, à des informations physiologiques précédemment stockées associées à une pluralité d'utilisateurs concernés ;
**caractérisé en ce que** les instructions comprennent en outre des instructions pour :
la détection (306) d'une proximité valide du dispositif de surveillance (102) avec le remède (104) consommé par l'utilisateur authentifié sur la base de l'intensité du signal associée à un ou plusieurs seconds signaux reçus en provenance du remède (104) ;
la détermination (308) du passage réussi du remède (104) dans le tube digestif de l'utilisateur sur la base d'une ou plusieurs signatures biométriques générées par le remède (104) lors du passage dans le tube digestif de l'utilisateur authentifié,
ledit processeur (112) étant configuré pour déterminer le passage réussi du remède (104) dans le tube digestif de l'utilisateur authentifié en exécutant les étapes de :
dérivation d'au moins l'une des informations d'accélération, de pH et de température du remède (104) provenant desdits un ou plusieurs seconds signaux pendant le passage du remède (104) dans le tube digestif de l'utilisateur authentifié,
détermination d'une ou plusieurs caractéristiques dont au moins une caractéristique à long terme, une caractéristique à court terme et une caractéristique péristaltique associée au tube digestif, sur la base des informations dérivées,
génération desdites une ou plusieurs signatures biométriques associées au tube digestif sur la base des caractéristiques déterminées,
détermination de la probabilité de passage du remède (104) dans le tube digestif de l'utilisateur authentifié sur la base desdites une ou plusieurs signatures biométriques,
comparaison de la probabilité de passage à une probabilité seuil préétablie, et
détermination du passage réussi (308) du remède (104) dans le tube digestif de l'utilisateur authentifié sur la base de la comparaison ; et
le signalement (310) de l'adhérence d'un médicament réussie de l'utilisateur authentifié sur la base de la détermination de la proximité valide du dispositif de surveillance (102) avec le remède (104) et du passage réussi du remède (104) dans le tube digestif de l'utilisateur authentifié.

5. Dispositif de surveillance (102) selon la revendication 4, ledit processeur (112) étant configuré pour détecter (306) la proximité valide du dispositif de surveillance (102) par rapport au remède (104) en exécutant les étapes de :
réception d'un ou plusieurs seconds signaux en provenance desdits un ou plusieurs seconds capteurs configurés dans le remède (104) ;
comparaison d'une valeur d'indicateur d'intensité de signal reçu de chacun desdits un ou plusieurs seconds signaux à une valeur seuil préétablie ; et
détermination que le dispositif de surveillance (102) est situé à une proximité valide du remède (104) consommé par l'utilisateur authentifié, sur la base de la comparaison à la valeur seuil préétablie.

6. Dispositif de surveillance (102) selon la revendication 4 ou 5, ledit processeur (124) étant en outre configuré pour activer le remède (104) en déclenchant un commutateur d'activation couplé au remède (104) avant la consommation du remède (104) par l'utilisateur.

7. Dispositif de surveillance (102) selon l'une quelconque des revendications 4 à 6, le processeur (112) étant en outre configuré pour :
créer un ou plusieurs profils d'utilisateurs pour lesdits un ou plusieurs utilisateurs concernés ;
obtenir un ou plusieurs signaux de test provenant du dispositif de surveillance (102) indiquant des informations physiologiques associées auxdits un ou plusieurs utilisateurs concernés lors de la consommation d'un placebo (104) ;
générer une ou plusieurs signatures biométriques de test associées au tube digestif desdits un ou plusieurs utilisateurs ;
déterminer une valeur de probabilité de test indiquant l'un du passage et du non-passage du placebo (104) dans le tube digestif desdits un ou plusieurs utilisateurs concernés ; et
mettre à jour lesdits un ou plusieurs profils d'utilisateurs avec les informations physiologiques correspondantes et la valeur de probabilité de test correspondant auxdits un ou plusieurs utilisateurs concernés.

8. Dispositif de surveillance (102) selon l'une quelconque des revendications 4 à 7, ledit dispositif de surveillance (102) étant configuré en tant que l'un parmi un dispositif portable, un dispositif portatif et un dispositif intégré.

9. Support non transitoire lisible par ordinateur sur lequel sont stockées des instructions qui, lorsqu'elles sont traitées par au moins un processeur (112), amènent un système (100) à exécuter le procédé (300) selon l'une quelconque des revendications 1 à 3.
